# EUROPEAN PATENT APPLICATION

(11) **EP 4 455 281 A1**
(43) Date of publication of application: **30.10.2024**
(21) Application number: 23169641.0
(22) Date of filing: 24.04.2023
(51) Int. Cl.: C12N 11/04, B09C 1/10

(54) **A METHOD FOR PREPARATION OF HYDROPHOBIC POROUS ALGINATE CARRIERS WITH INCORPORATED MICROORGANISMS**

(71) Applicant: Jozef Stefan Institute, 1000 Ljubljana (SI)
(72) Inventor: Zugan, Maja, 1000 Ljubljana (SI); Rybkin, Iaroslav, 1000 Ljubljana (RU); Rijavec, Tomaz, 1000 Ljubljana (SI); Lapanje, Ales, 1000 Ljubljana (SI)
(74) Representative: Patentni Biro AF d.o.o.

(57) **Abstract**

The present invention belongs to the field of biotechnology, more precisely to the field of method for preparation of products containing incorporated microorganisms. The invention relates to a method for preparation of hydrophobic porous alginate carriers containing incorporated microorganisms and alginate carriers with incorporated microorganisms prepared with said method.

## Description

### Field of the invention

The present invention belongs to the field of biotechnology, more precisely to the field of method for preparation of products containing incorporated microorganisms. The invention relates to a method for preparation of hydrophobic porous alginate carriers with incorporated microorganisms and to hydrophobic porous alginate carriers with incorporated microorganisms prepared with said method. One aspect of the invention also relates to use of said hydrophobic porous alginate carriers.

### Background of the invention and the technical problem

In general, bacterial attachment to various carriers, made of organic or inorganic materials, is used in different biotechnological processes, such as wastewater treatment plants and soil bioremediation, antibiotic and antibody production, in food industry, and in pharmacy such as drugs production. Carriers used in bioremediation processes with microbial bioaugmentation (MB) harbour cells and enable (1) prolonged use of cell biomass, (2) protection of cells from harsh environmental conditions, (3) decreasing microbial competition with autochthonous microbiota as well as prevent protozoan grazing of introduced microbial biomass and (4) providing a stable local environment (pH, ionic strength, nutrients).

Specifically, the bioremediation of xenobiotics is problematic due to the dispersed and low local concentrations of contaminants and consequently the remediation using microbes is currently at low efficiency since huge amount of microbial biomass needs to be introduced. According to current state in the field of bioremediation, three major aspects can be improved in the remediation strategies in order to increase efficiency of bioremediation, namely:
1. Increase of the local pollutant concentration: concentration of the pollutant should be locally increased in order to increase physiological responses of bacteria (van Tatenhove-Pel et al., 2021; doi: 10.1038/s41396-020-00806-9),
2. Longer exposure times: the increased concentration of pollutants must be provided to microbial cells for longer time since most of the pollutants are recalcitrant for degradation and microbial processes are accordingly slow,
3. Redistribution of pollutant: even if the pollutants are present in higher concentration they are distributed in patches. Accordingly, there is relatively low probability of introduced microbial biomass or consortia that will get distributed in the vicinity of pollutant patches. Therefore, it is needed to help either cells or microbes to get closer to the patches through the carrier manipulation or to distribute pollutant closer to microbes.

In bioremediation techniques with MB, the material that is used for making carriers should possess the following properties:
(i) enabling higher partition coefficients for the particular pollutant to absorb it,
(ii) no toxicity for the microorganisms, and
(iii) biodegradability.

Although, there are many reports on preparation of different hydrophobic matrices with designing hydrophobic hydrogels in terms of drug delivery (Takei et al., 2020, doi: 10.1016/j.ijbiomac.2020.01.227; Yao et al., 2010, doi: 10.1007/s00449-009-0349-2; Ye at al., 2019, doi: 10.1155/2019/9170732) by using ethyltrimethoxysilane, octylamine and polybutyl, the alginate with modified properties would be the superior to the other hydrogels since it is naturally available, is biodegradable and is not toxic to biota.

In this relation, the hydrophilic Ca²⁺ crosslinked alginate-based carriers have been described for cell immobilisation in documents US 5766907A, US5073491A and US4927761A, EP0784651B1). The alginate hydrogels are suitable for cell immobilisation due to liquid retaining properties, rigidity, density and structure. All of these parameters can be controlled by altering the concentration of alginate and the type and concentrations of cation used. Although usually used alginate matrix crosslinked with Ca²⁺ can fulfil the above-mentioned criteria for hydrophilic pollutants, this matrix is not appropriate for hydrophobic pollutants.

Therefore, there is a need for hydrophobic alginate carriers for use in bioremediation and the approaches for modifying alginate based carriers bearing microorganism to become hydrophobic are focused on the surface modification of the carriers using hydrophobic polymers or other materials and not the modification of the alginate itself (Jianqiang Shi et al 2020; IOP Conf. Ser.: Mater. Sci. Eng. 729 0)

Since the regular form of alginate is hydrophilic, it requires to be treated to become hydrophobic. It has been shown that introduction of ZrOCl₂ or Zr²⁺ ions instead of Ca²⁺, in alginate matrix alters surface properties and makes the matrix hydrophobic (Wang et al. 2019; doi: 10.1016/j.jcis.2018.08.073)). Hence, the Zr²⁺ salts can change alginate to obtain hydrophobic properties, that enables adsorption of hydrophobic substrates due to the differences in partition coefficients. This results in an increase of the local concentration of the hydrophobic substances that result in increased efficiency of microbes to degrade or transform such substrates. As an example, this can be applied in stimulated bioremediation of PAH polluted environments. However, in contrast to the Ca²⁺ based gelation of the alginate, the procedure that is used in preparation of hydrophobic alginate results in highly acidic, extremely hydrophobic and nonporous matrix that does not enable efficient exchange of water-soluble nutrients. As such the local environment is highly toxic for microorganisms and accordingly not suitable for MB.

If the hydrophobic alginate is becoming porous by other way than it is currently used it enables direct contact with the nutrients on one way and with the substrate, the pollutant, on other. The porosity of alginate can be achieved in various ways. For an example in the patent US11028247B2, the use of CaCO₃, that is mixed along with sodium alginate is described. The prepared mixture was put in an autoclave, where it was pressurised with gaseous carbon dioxide up to 50 bars at room temperature. Further on, hydrogels were dried in a high-pressure autoclave at 120 bar pressure of CO₂ atmosphere. However, due to the laborious and time-consuming procedure as well as pore size smaller than cell size, the method of preparation and the matrix are both unsuitable for use as cell carriers in bioremediation or other environmental and biotechnological applications. Patent AU776009B2 describes a much more suitable method for producing porous alginate hydrogels, which is achieved by the release of gas from the reaction of acid and base.

The present invention addresses the technical problem of optimization of known processes for preparation of hydrophobic porous alginate carriers that will not be lethal to the microbes. The aim of the invention is to provide an efficient and reliable method for the preparation of said hydrophobic alginate carriers with incorporated microorganisms suitable for use in various applications, particularly in bioremediation.

### Prior art

Patent US8252317B2 describes novel metal alginates prepared from non-cross-linked alginate monomers such as sodium alginate and an aqueous solution of a group 4, 5, or 6 metal oxyhalide. The novel group 4, 5, and 6 metal alginates are useful in the preparation of cell culture supports, exhibit useful X-ray contrast properties, and exhibit unanticipated stability to standard autoclave conditions relative to known metal alginates such as calcium alginate. In general, the novel metal alginates provided by the present invention offer features and properties not observed in known metal alginates such as calcium or barium alginates. In this solution, Zr²⁺ ions with sodium alginate were used to increase thermal stability of alginate as a support material for cultivation of eukaryotic cells and not as an encapsulation substrate, since cells by such method cannot be entrapped inside this matrix.

Patent US8252317B2 suggests use of Zr²⁺ ions for sodium alginate crosslinking and gelation in order to alter the final physicochemical properties of the matrix such as better heat stability and suitability in x-ray imaging.

In addition, various techniques of hydrophobic alginate preparation in terms of oil absorption as a physical way of oil removal from oil spill pollution sites were proposed in different scientific articles by Yang et al., 2012 (doi: 87. 10.1016/j.carbpol.2011.09.046), Yang et al., 2021 (doi: 10.1021/acs.langmuir.0c03229) and in patent CN108976468B. These methods differ from the present invention.

The procedure for the preparation of the porous hydrophobic alginate carriers using Zr²⁺ ions is very harsh to microbes (low pH, freeze thawing or high temperatures) and they cannot survive it. The procedure is deleterious to the microorganisms due to:
- toxicity of ZrOCl₂ can be attributed either to Zr²⁺ ions or low pH when alginate is treated with Zr salts,
- low accessibility of nutrients dispersed in aqueous phase, since the matrix is exposing a hydrophobic surface inhibiting diffusion of water and consequently the nutrient flux is expected to be stalled,
- porous hydrophobic alginate is prepared by the method that implies freeze thawing or elevated temperatures above the point of physiological compensation by microbes.

Therefore, the carrier must be prepared in such a way that it is not toxic and provides an initial source of nutrients. The present invention aims to address this problem.

### Description of the invention

Although preparation of hydrophobic alginate as well as introducing porosity is well documented, especially in terms of drug delivery, preparation of hydrophobic porous alginate as a carrier for bacterial immobilisation for the purpose of oil remediation has never been sufficiently optimized due to the above-mentioned technological limitations. The technical problem is solved as defined in the independent claims, whereas preferred embodiments are defined in dependent claims.

The method for preparation of hydrophobic porous alginate carriers with incorporated microorganisms according to the invention comprises treatment of alginic acid or its salts with monovalent cations (e.g. Na-alginate, K-alginate) with a combination of compounds from which HCO₃¹⁻ and CO₃²⁻ions can be released, divalent cations such as Ca²⁺, Ba²⁺, Cu²⁺, Sr²⁺, Zn²⁺, Fe²⁺, Fe³⁺ Mn²⁺, Al³⁺, ions and treating gel with the Zr⁴⁺ ions either simultaneously as the induction of gelation or after the gelation. Said combination of ions ensures more suitable conditions for survival of microorganisms and suitable carrier properties with sufficient porosity and surface hydrophobic properties. Furthermore, the methods are performed under standard conditions.

In preferred embodiments, divalent Ca ions originate either from CaCO₃, CaCl₂ or any other compound releasing said Ca ions, wherein Zr ions may originate from ZrOCl₂ or any other compound releasing Zr ions. Said sources of ions may also be used in combinations, for example CaCOs and CaCl₂.

In a preferred embodiment, said treatment of alginic acid is step-wise, wherein addition of Zr ions releasing compound is performed after treatment with Ca²⁺ releasing compound.

Microorganisms and nutrients may be added to the alginic acid prior to the treatment with Ca and Zr ions or post treatment, i.e., after preparation of alginate carriers is finalized. In case of the latter, a suitable microbial suspension is added to the prepared carriers and vacuum is used to impregnate the carriers with microbes. Optionally, after application of vacuum a further step of treatment with CaCl₂ is performed to ensure gelation of alginate trapped within the pores of the carrier. Populations and consortia of microbes are used in the group of Gram negative and Gram positive, mesophilic, thermophilic, psychrophilic aerobic and anaerobic as well as halotolerant, nonhalophiles as well as slight halophiles bacteria as well as fungi.

According to the first exemplary embodiment, the method comprises the following steps:
- mixing alginic acid and CaCO₃,
- dropwise adding the mixture obtained in the previous step to ZrOCl₂ to introduce pores and transforming alginate surface from hydrophilic to hydrophobic,
- washing with a buffer that can buffer pH ranges between 4 to 10.

Addition of microorganisms and nutrients can be performed before addition of ZrOCl₂ or after washing with the buffer.

In this embodiment, alginate and CaCO₃ are not pre-gelated with Ca²⁺ ions prior to Zr treatment. Here, the acidity of ZrOCl₂ decrease pH and release Ca²⁺ ions from CaCOs that are then competing with Zr⁴⁺ ions for cross-linking alginate polymers and together forming hydrophobic and porous alginate. After this procedure the alginate molecules can be additionally cross-linked in CaCl₂ solution if it is necessary to obtain stronger gels.

According to the second exemplary embodiment, the method comprises the following steps:
- mixing alginic acid and CaCO₃,
- dropwise adding the mixture obtained in the previous step into CaCl₂ to obtain gelated alginate particles, for example formed as beads, sheets, threads,
- adding ZrOCl₂ to introduce pores and transforming alginate surface from hydrophilic to hydrophobic,
- washing with a buffer that can buffer pH ranges between 4 to 10.

Addition of microorganisms and nutrients can be performed before addition of CaCl₂ or after washing with the buffer.

CaCOs in formed alginate beads or in any other forms of alginates, including as a solution impregnating solid porous carrier is then reacting with acidic ZrOCl₂ solution. In this case CaCOs does not only provide the indirect production of pores, but by neutralising acidity through the CaCOs buffering capacity, protects viable bacterial cells from toxic effects of acidic ZrOCl₂ solution. The incubation times of carriers in ZrOCl₂ solution may be adjusted, according to the presence of bacterial cells in sodium alginate and CaCOs in the suspension. After exposure to the Zr salts the treatment is stopped by the addition of phosphate buffer. The time of the exposure also determines size of pores that are formed within the gelated alginate.

According to the third exemplary embodiment, the method comprises the following steps:
- mixing alginic acid and CaCO₃,
- adding solid support carriers,
- dropwise adding the mixture obtained in the previous step into CaCl₂ to obtain gelated alginate particles, for example formed as beads, sheets, threads,
- adding ZrOCl₂ to introduce pores and transforming alginate surface from hydrophilic to hydrophobic,
- washing with a buffer that can buffer pH ranges between 4 to 10.

Addition of microorganisms and nutrients can be performed before addition of CaCl₂, either separately or together with addition of the solid support carriers, or after washing with the buffer. Solid support carriers may be selected in the group comprising expanded clay, porous sandstones, vermiculite, straw particles, cellulose, and similar. CaCOs in formed alginate beads or in any other forms of alginates, including as a solution impregnating solid porous carrier is then reacting with acidic ZrOCl₂ solution. In this case CaCOs does not only provide the indirect production of pores, but by neutralising acidity through the CaCOs buffering capacity, protects viable bacterial cells from toxic effects of acidic ZrOCl₂ solution. The incubation times of carriers in ZrOCl₂ solution may be adjusted, according to the presence of bacterial cells in sodium alginate and CaCOs in the suspension. After exposure to the Zr salts the treatment is stopped by the addition of phosphate buffer. The time of the exposure also determines size of pores that are formed within the gelated alginate.

According to the fourth exemplary embodiment, the method comprises the following steps:
- mixing alginic acid and CaCO₃,
- dropwise adding the mixture obtained in the previous step into CaCl₂ to obtain gelated alginate particles, for example formed as beads, sheets, threads,
- adding ZrOCl₂ to introduce pores and transforming alginate surface from hydrophilic to hydrophobic,
- washing with a buffer that can buffer pH ranges between 4 to 10 to obtain alginate carriers,
- adding microorganisms and nutrients to the alginate carriers prepared in the previous step,
- treatment with vacuum to impregnate the carriers with the microorganisms added in the previous step, and
- optionally adding CaCl₂ for improved gelation.

According to this embodiment, porous alginate matrix is prepared in advance, wherein alginate is made porous with different harsh methods and is crosslinked with Ca²⁺ ions as well as treated with solution containing Zr⁴⁺. After that the bacterial cells are introduced using depressurisation methods such as those known from patent SI24910A.

According to the fifth exemplary embodiment, the method comprises the following steps:
- mixing alginic acid and CaCO₃,
- dropwise adding the mixture obtained in the previous step into CaCl₂ to obtain gelated alginate particles, for example formed as beads, sheets, threads,
- adding an acid solution, such as acetic acid, lactic acid, citric acid, orthophosporic acid, or preferably HCl, for acidification,
- adding ZrOCl₂ to introduce pores and transforming alginate surface from hydrophilic to hydrophobic,
- washing with a buffer that can buffer pH ranges between 4 to 10 to obtain washed alginate carriers,
- adding microorganisms and nutrients to the alginate carriers prepared in the previous step,
- treatment with vacuum to impregnate the carriers with the microorganisms added in the previous step, and
- optionally adding CaCl₂ for improved gelation.

According to this embodiment, porous alginate matrix is prepared in advance, wherein alginate is made porous with different harsh methods and is crosslinked with Ca²⁺ ions as well as treated with solution containing Zr⁴⁺. After that the bacterial cells are introduced using depressurisation methods such as those known from patent SI24910A.

The methods according to the invention enable synthesis of novel alginate hydrogel for use as a bacterial carrier for removal of hydrophobic pollutants such as oil. The carriers according to the invention can be used in bioremediation of water and soil as they exhibit all three crucial properties:
- hydrophobicity of alginate surface,
- porosity of the matrix and
- bacterial confinement in the carrier matrix.

### Brief description of the drawings

The present invention will be further described based on detailed exemplary embodiments, examples, and figures, which show:
- Figure 1: Schematic representation of preparation of hydrophobic porous alginate carriers with confined bacteria using different approaches
- Figure 2: Schematic representation of hydrophobic porous alginate carrier cross-section of hydrophobic porous alginate carrier with immobilised bacteria prepared by method 1 (figure 2a), method 2 (figure 2b) and method 3 (figure 2c) as described below
- Figure 3: Minimal inhibitory test of ZrOCl₂ in *Escherichia coli* in nutrient broth (NB) media. *E. coli* cells (OD₆₀₀ₙₘ=0.1) expressing emGFP through promoter leakage were exposed to concentrations of Zr salts from 0.16% to 1.25% in two-fold steps series dilutions (see legend). In the control group Zr salts were omitted. The graph is showing the growth curves following emGFP fluorescence intensities monitored at the 485nm excitation and 528nm emission wavelengths

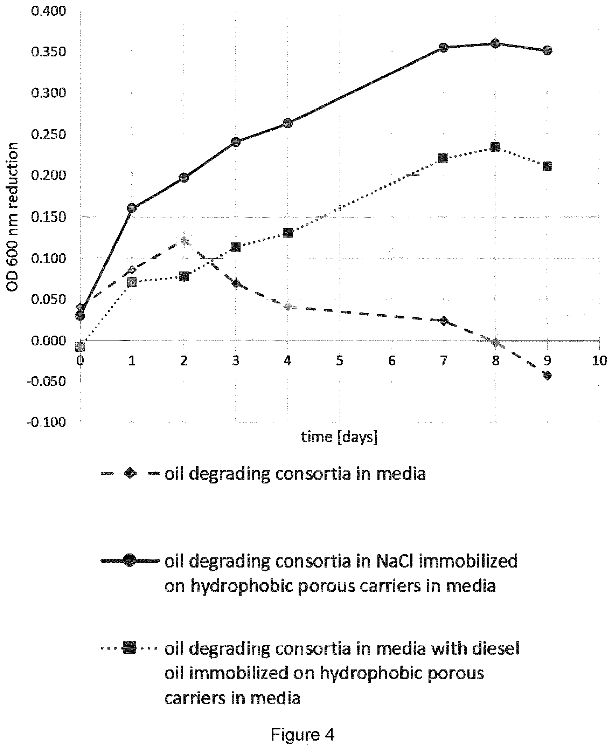

- Figure 4: Oil degradation efficiencies of immobilized and free-living oil degrading bacterial consortia. The oil degradation is monitored by the reduction of 2,6-dichlorophenolindophenol (DCPIP), which is equivalent to enzymatic degradation of oil by bacteria. Three different scenarios were tested: (i) oil degrading consortium immobilised on carriers in minimal media with diesel oil, (ii) oil degrading consortium immobilised on carriers in 0.9 % NaCl and (iii) oil degrading consortium inoculated in liquid media. Higher light absorption values of tested supernatants at OD₆₀₀ₙₘ represent higher removal efficiencies
- Figure 5: Microscopic picture of carriers with a diameter of less than 30µm and prepared on a basis of method 1 as described below.

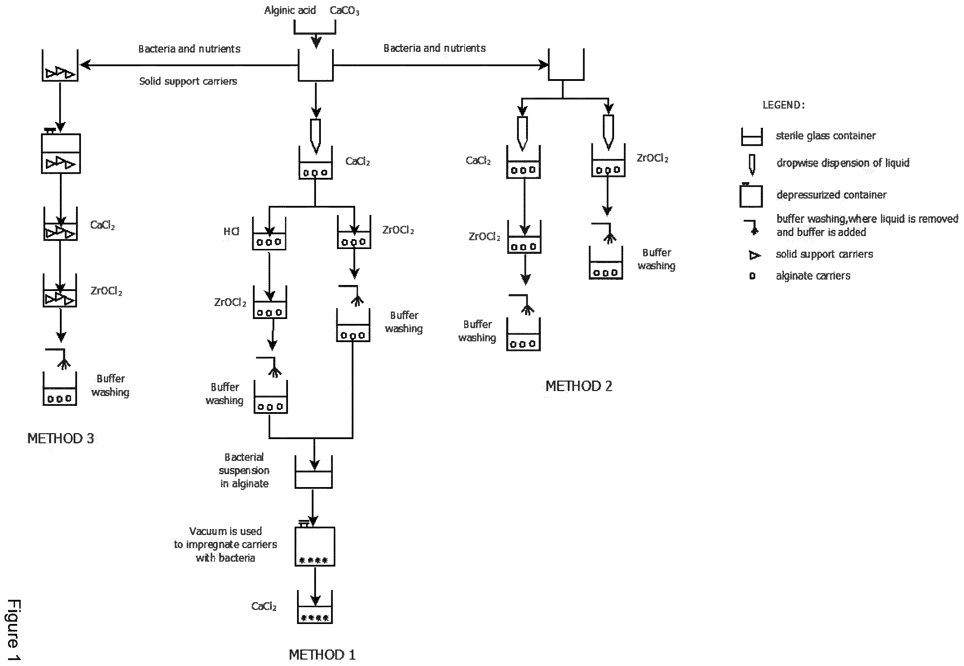

The methods according to the invention may be performed in various combinations, wherein some preferred examples have been described above. Figure 1 shows a schematic representation of preparation of hydrophobic porous alginate carriers with confined bacteria using different approaches.

For all methods, a solution of sodium alginate and calcium carbonate is prepared. Aseptic conditions are maintained throughout the experiment and all solutions are sterilised prior to their use either by autoclaving or filtration. The optimal alginate concentration in distilled water was found to be 4 %, but concentrations from 0.5 % to 5 % can be used. Alginate is gradually added to water, preferably heated water (temperatures from 18°C to 50°C), to ensure adequate solubilization. Afterwards, 0.1 to 10%, most preferably 1 % w/v CaCOs with particle sizes in the range from 50 nm to 10 µm is used as a source of HCO₃¹⁻ ions. However, other divalent soluble salts might be used. To ensure calcium carbonate was properly dispersed in alginate mixture, firstly ultrasonic bath is used for a time period of 5 min to 30 min at 40 Hz to 75 Hz, preferably for 15 min at 60 Hz. Then the mixture is well mixed using a magnetic stirrer at 1300 RPM and heated between 50°C to 95°C.

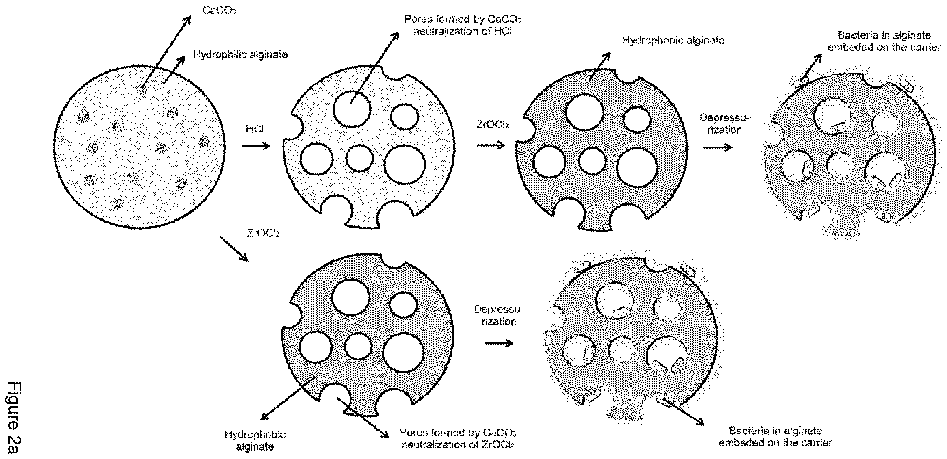

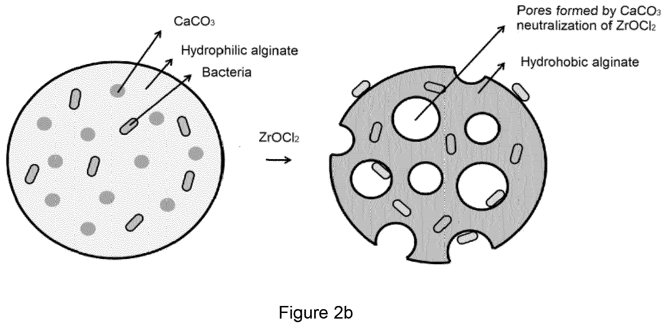

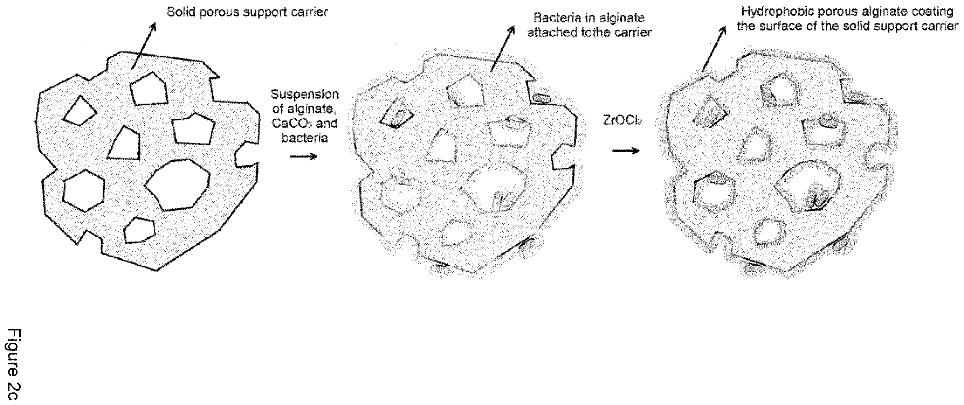

After this step, microorganisms may be added together with nutrients. However, they can be added at a later stage as illustrated in Figure 2. Detailed exemplary embodiments of the method according to the invention will be described based on figures 1 and 2.

### Method 1: Separate preparation of hydrophobic carriers and consequent addition of microbial cells

This method comprises the following steps:
a) Sterile suspension of 0.5% to 4% alginate with 0.1 to 10% and sizes 50 nm - 10 µm insoluble calcium carbonate solution is added dropwise to 0.1 M to 10 M, preferably 1 M CaCl₂ solution for a minimum of 5 minutes to form beads, sheets, threads etc. Concentrations of CaCl₂ can vary, but are always in excess to the alginate concentration and volume. Since proper alginate gelation is achieved in the presence of divalent ions, soluble salts of divalent ions can be used, for example magnesium and barium chlorides in the same concentration ranges as CaCl₂. After gelation is finished, the solution of CaCl₂ is discarded, and beads are washed using distilled water no less than two times in two to five times the volume of the alginate carriers.
b) Optionally, once the beads are formed, acid in the form of 0.1 M up to 1 M, preferably 0.5 M HCl is added to introduce the pores in a dense alginate matrix. Furthermore, HCl can be substituted by other acid solutions, such as acetic acid, lactic acid, citric acid, orthophosporic acid, which can induce porosity. Incubation of the beads in HCl solution largely depends on the size of the beads, but 1 to 30 minutes is required. The process is finished once all CaCOs particles are dissolved. After incubation, acid needs to be removed completely from the beads by washing with two to four times the volume of the beads using 0.1 M phosphate buffer or other buffers that can buffer pH ranges between 4 to 10. Washing should be repeated until the optimal pH range for growth of particular microbial cells is achieved.
c) Addition of 1% to 10 % ZrOCl₂ solution and left to incubate for at least 10 minutes, preferably 15 minutes. The hydrophobicity of the alginate carriers depends on the concentration of the Zr²⁺ ions in the solution, so concentrations less than 1 % and incubation times lower than 10 minutes are not recommended, since low hydrophobicity will be achieved. Treatment with Zr salts helps to achieve hydrophobicity and porosity of alginate matrix. The acidity of 1 % to 10 % w/v ZrOCl₂ can cause dissolution of CaCOs embedded particles in the matrix as well. In this case, ZrOCl₂ is added after alginate gelation that is caused by addition of CaCl₂. Carriers should be incubated not les than 30 min and not more than 3 h in ZrOCl₂ to dissolve all CaCOs and to make pores. Final washing with buffer of desired pH is needed that should be repeated for more than 3 times. Since carriers are prepared aseptically by using all sterile solutions and material, no sterilisation of formed carriers is needed.
d) Once carriers are prepared, bacterial cells can be immobilised on the matrix. Bacterial cells are selected in the group comprising negative and Gram positive, mesofilic, thermophilic, psychrophilic aerobic and anaerobic as well as halotolerant, nonhalophiles as well as slight halophiles, and also bacteria sustaining the pH ranges from 4 to 10 (slight acidophiles to alkaliphiles). The cell biomass of the selected bacterial strain is collected, washed and combined with 1 % alginate in suitable medium where divalent cations are omitted in order to prevent gelation. Different concentrations of alginate and amount of bacteria can be used, from 0.5 % to 3 %, and from OD₆₀₀ = 0.1 to 5, respectively. Prepared hydrophobic porous alginate carriers are submerged in solutions containing alginate and bacteria in sterile flasks. A vacuum pump is then used for degassing the suspension and remove the air in previously prepared porous carriers (see a and b). The vacuumization should reach between 10 % to 90 % of the atmospheric pressure, which is used to substitute gasses in pores of carriers with the surrounding liquid containing bacterial cells. Alternating the vacuuming process and pressure release is performed up to 5 times or until no air bubbles are observed in the liquid. Leftover liquid of alginate and bacteria is removed and solution of divalent cations (Ca²⁺) is added. Attachment of bacteria inside the pores is achieved through alginate gelation, using 0.1 M to 10 M, preferably 1 M CaCl₂.

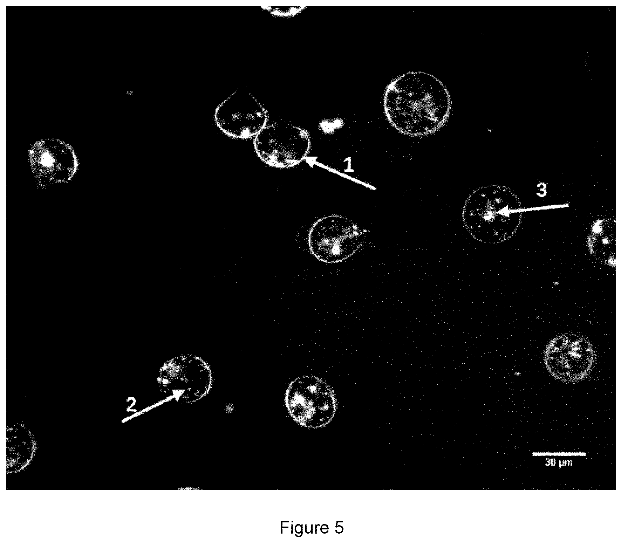

As shown in figure 5 this method enables formation of small beads of the 30µm size in diameter (see arrow 1) using microfluidic approach of formation of hydrophobic beads during the incubation in ZrClO₂ solution after pores are introduced (see arrow 2) using the HCl treatment. The pores in these small beads are filled with the hydrophilic alginate containing cells (see arrow 3) by applying the vacuuming impregnation procedure.

### Method 2: Preparation of hydrophobic carriers by embedding microbial cells

This method comprises the following steps:
a) Bacterial cells and optionally nutrients are added in the suspension of alginate and 0.1% to 10% of 10 nm - 10 µm particle sizes CaCOs before the treatment with ZrOCl₂. Here bacteria are washed with concentration of OD₆₀₀ between 0.1 to 5 and then mixed with the sodium alginate solution comprising CaCOs in prior to the gelation.
b) Sodium alginate/CaCO₃ suspension combined with bacteria is further treated in the same manner as described in the previous method - solution is dropwise added to 0.1 M to 1 M CaCl₂ where beads or other sorts of shapes that can be formed during this process are then left to gelate for 15 minutes. This is then followed by the incubation for 5 to 60 minutes in 1 % to 10 % ZrOCl₂ solution and then washed two times with phosphate or other suitable buffers that are keeping optimal physiological pH between pH 4 to pH 10 for immobilized bacteria or consortia.
c) Alginate containing cells and CaCO₃ particles can be directly added into the 1 % to 10 % ZrOCl₂ solution and incubated for 5 to 60 minutes instead of using treatment described under b). To obtain needed strength of the matrix it can be additionally gelated by soaking in 0.1 M to 1 M CaCl₂ solution the formed carriers. The pH is controlled by washing as it is described in b).

### Method 3: Preparation of hydrophobic carriers by impregnation of solid porous material with matrix containing embedded microbial cells

This method comprises the following steps:
a) A suspension comprising bacterial cells, nutrients, alginate and 0.1% to 10% CaCO₃ with 10 nm - 10 µm particle sizes is used for impregnation of porous solid supports, such as expanded clay, porous sandstones, vermiculite, straw particles, cellulose, etc., using depressurization method described in Method 1.
   In this suspension nutrients can also be added.
b) Such impregnated solid support can be either (i) gelated with solution of CaCl₂ concentration between 0.1 M to 10 M, then washed in water and at the end treated with 1 % to 10 % solution of ZrOCl₂ as described in method 1. c). or (ii) CaCl₂ gelation can be omitted and instead carriers can be directly dropped in or washed in 1 % to 10 % ZrOCl₂ solution.
c) At the end pH must be adjusted by washing and incubation of carriers in phosphate or other suitable buffers.

### Examples

### Example 1. Preparation of carriers using method 1 as described above

The alginate carriers comprising bacterial cells were prepared as follows:
1. Alginic acid sodium salt, from brown algae (Sigma Aldrich, United States of America) was gradually dissolved in distilled water in the final concentration of 4 % w/v. To facilitate dissolution process, magnetic stirring (1500 rpm) and heating (90 °C) was applied. After the sodium alginate is dissolved, CaCOs was added to obtain final concentration of 1 % w/v. Sodium alginate and CaCOs mixture was incubated in an ultrasonic bath, heated to 80 °C for 20 minutes. Once dissolved, 4 % w/v sodium alginate and 1 % w/v CaCOs suspension was sterilised using autoclave at 121 °C for 15 minutes. 0.5 M HCl solution was prepared by diluting 37 % v/v HCl (Sigma Aldrich, United States of America) accordingly which were then filter sterilised using PES 0.2 µm filters. ZrOCl₂ × 8 H₂O (Alfa Aesar, United States of America) was prepared by dissolution in distilled water reaching final concentration of 5 % w/v and sterilized by autoclaving at 121 °C for 15 minutes. 1 M CaCl₂ solution was prepared by dissolving an appropriate amount of CaCl₂ in distilled water and then sterilised by autoclaving, as mentioned previously.
2. 10 mL of 1 M CaCl₂ was poured in a sterile 50 mL tube and 4 % w/v sodium alginate containing 1 % w/v CaCO₃ solution was added to the CaCl₂ solution in a dropwise manner. After 15 min of incubation, carriers were removed from1 M CaCl₂ and washed using sterile distilled water (2 times). By incubating carriers for 20 minutes in 0.5 M HCl the CaCOs core was dissolved. After intensive washing with sterile 0.1 M phosphate buffer pH=7 (4 times in 2x volume), 5 % ZrOCl₂ was added and left for 15 min to obtain hydrophobic alginate matrix. Carriers were then extensively washed with sterile 0.1 M phosphate buffer pH=7 (4 times in 2x volume) until all ZrOCl₂ is removed from the carriers and obtaining neutral pH.
3. Bacterial biomass of oil degrading bacterial consortia suspended in the minimal media and supplemented with diesel oil (2 mL/L) (OD₆₀₀ = 1) was mixed with 1 % w/v alginic acid. This mixture was then poured over the previously prepared porous hydrophobic carriers. Vacuum pump was used to degas carriers at conditions below the 90% of the atmospheric pressure. By vacuumisations the mixture was impregnating the porous carriers. Remaining liquid was discarded after vacuumization and carriers were transferred to 1 M CaCl₂ solution for 15 min. After this incubation, carriers were washed by distilled water.

Both methods for preparation of pores, namely, the HCl pre-treatment and direct treatment by ZrOCl₂, were then tested for the differences in efficiencies of the uptake of microbial cell by vacuumization method. The method of the dissolution of CaCOs particles trapped in the alginate matrix with ZrOCl₂ showed 1.67 times higher loading capacity of microorganisms than the method where HCl is used (Table 1 below).

**Table 1: Loading capacity of microorganisms**

| HCl dissolution of CaCO3 for 15 minutes | | | | | |
|---|---|---|---|---|---|
| weight before vacuum (g) w | eight after vacuum (g) | difference | gig carriers | average | sd |
| 0.86 | 0.94 | 0.08 | 0.09 | 0.12 | 0.03 |
| 0.85 | 0.83 | -0.02 | -0.02 | | |
| 0.87 | 1 | 0.13 | 0.15 | | |
| | | | | | |

| ZrOCl2 dissolution of CaCO3 for 60 minutes | | | | | |
|---|---|---|---|---|---|
| weight before vacuum (g) weight afte | r vacuum (g) | difference | g/g carriers | average | sd |
| 1 | 1.19 | 0.19 | 0.19 | 0.20 | 0.03 |
| 1.01 | 1.25 | 0.24 | 0.24 | | |
| 1 | 1.16 | 0.16 | 0.16 | | |

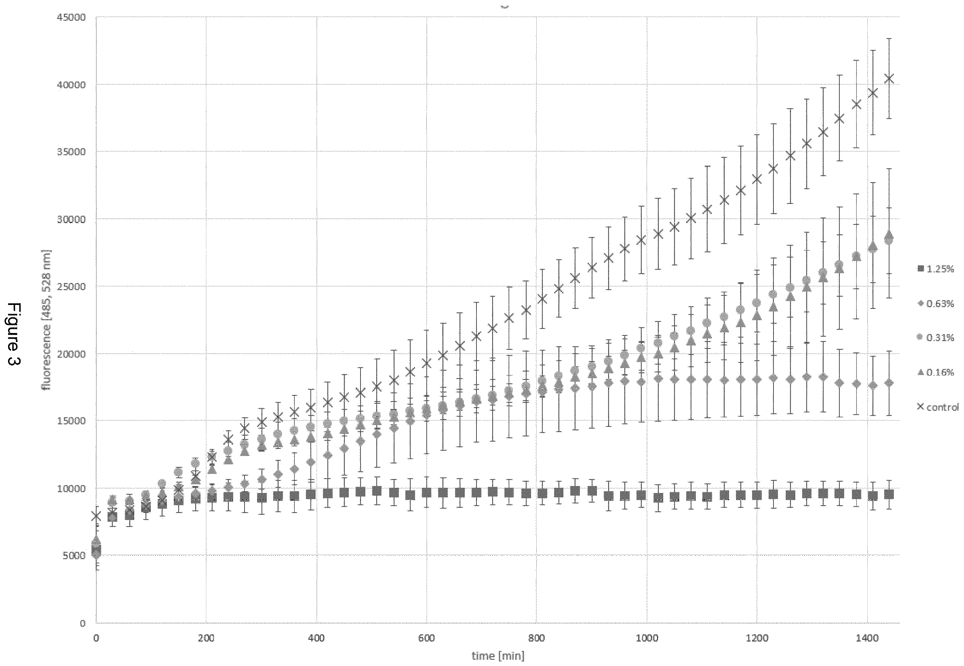

After preparation of carriers the toxicity of different concentrations of ZrOCl₂ through the inhibition of *Escherichia coli* TOP10 expressing GFP was determined by measurement of fluorescence (Fig.3).

Minimal inhibitory test of ZrOCl₂ in *Escherichia coli* in nutrient broth (NB) media. *E. coli* cells (OD600nm=0.1) expressing emGFP through promoter leakage were exposed to concentrations of Zr salts from 0.16% to 1.25% in two-fold steps series dilutions (see legend). In the control group Zr salts were omitted. The graph is showing the growth curves following emGFP fluorescence intensities monitored at the 485 nm excitation and 528 nm emission wavelengths. The results suggest that concentrations below 1% allow growth of *E*. *coli.*

### Example 2: Preparation of carriers using method 2 as described above with addition of bacterial cells in the last step

The alginate carriers comprising bacterial cells were prepared as follows:
1. Mixture of 1 % w/v alginic acid sodium salt and 1 % w/v CaCOs was added in a dropwise manner to 1 M CaCl₂ and carriers were washed with sterile dH₂O in the same manner as described in Example 1.
2. 5 % ZrOCl₂ was added and left for 60 min to dissolve CaCOs. Carriers were then extensively washed with sterile 0.1 M phosphate buffer pH=7 (4 times in 2x volume) until all ZrOCl₂ was removed from the carriers and the pH became neutral.
3. Oil degrading bacterial consortia were introduced into the prepared carriers in the same manner as described in Example 1, paragraph 3.

### Example 3: Preparation of carriers using method 2 as described above with addition of bacterial cells prior to gelation

The alginate carriers comprising bacterial cells were prepared as follows:
1. Mixture of 1 % w/v alginic acid sodium salt, 1 % w/v CaCOs and oil degrading consortia at cell densities of OD₆₀₀ₙₘ = 1 in 0.9 % NaCl is added to 1 M CaCl₂ in a dropwise manner and left to incubate for 15 minutes. Afterwards, carriers were washed with sterile dH₂O.
2. Carriers were transferred to 1 % ZrOCl₂ solution where they are incubated for 40-minutes. 0.1 M phosphate buffer with pH = 7 is used for washing ZrOCl₂ solution. Washed carriers are stored in 0.1 M phosphate buffer pH = 7.

### Example 4. Preparation of carriers using method 2 as described above with addition of bacterial cells and nutrients prior to gelation

The alginate carriers comprising bacterial cells were prepared as follows:
1. Suspension of alginic acid, CaCOs and bacterial biomass was prepared in the same manner as described above (Example 3), but nutrients are added in as well. In this example the minimal medium is providing nutrients required for the growth of oil degrading bacterial consortia. Final suspension contained 4 % alginate w/v, 1 % CaCO₃, bacterial biomass with OD₆₀₀ = 1 and nutrients in concentrations described below. Carriers were formed in the same manner as previously described (Example 3) using Zr salts for dissolution of CaCOs.

Preparation and composition of minimal media (MM) used for cultivation of oil degrading bacterial consortia: 1ml autoclaved PAS salts and 1-2 ml/l of filtered diesel fraction is added to the 500 ml autoclaved MM.

### MM composition:

- 1 g/L K₂HPO₄
- 0.5 g/L KH₂PO₄
- 0.1 g/L MgSO₄ x 7H₂O
- 0.1 g/L NaCl
- 1 g/L (NH₄)₂SO₄
- 0.005% (wt/vol) Yeast extract

### 100 x PAS salts:

- 19.5 g/L MgSO₄
- 5 g/L MnSO₄ × H₂O
- 1 g/L FeSO₄ × 7H₂O
- 0.3 g/L CaCl₂ × 2H₂O
- several drops of concentrated H₂SO₄ per L added post autoclaving to prevent precipitation of basic salts.

### Example 5. Preparation of carriers using method 3 as described above

The alginate carriers comprising bacterial cells were prepared as follows:
1. Suspension of alginic acid, CaCO₃, bacterial biomass and nutrients was prepared as described in Example 4.
2. Sterile activated clay solid support material was submerged in the prepared solution and depressurized as described above. Remaining liquid after depressurization was discarded and clay carriers were transferred to 1 M CaCl₂ solution for 15 min, washed with distilled water and then subjected to treatment with 5 % ZrOCl₂ solution for 40 min. At the end carriers were washed three times with 0.1 M phosphate buffer using volumes 2 times larger than the volume of the volume of the impregnating suspension.

Oil degrading activity of immobilized oil degrading bacterial consortia isolated from an oil polluted environment was determined, wherein polyaromatic hydrocarbons (PAH) as the only carbon source together with DCPIP as indicator of metabolic activity were added (Fig.4). The oil degradation is monitored by the reduction of 2,6-dichlorophenolindophenol (DCPIP), which is equivalent to enzymatic degradation of oil by bacteria. Three different scenarios were tested: (i) oil degrading consortium immobilised on carriers in minimal media with diesel oil, (ii) oil degrading consortium immobilised on carriers in 0.9 % NaCl and (iii) oil degrading consortium inoculated in liquid media. Higher light absorption values of tested supernatants at OD600nm represent higher removal efficiencies. Results presented in the figure 4 indicate that the impregnation of the carriers with different bacteria representing oil degrading consortium embedded in the hydrophobic alginate prolong the activity of the cells at least for 9 days of the test with the maximal degradation level reaching 4.5 times higher than the maximal degradation level of the oil with consortia that were not introduced in the carriers.

## Claims

1. A method for preparation of hydrophobic porous alginate carriers with incorporated microorganisms, said method comprising
- treatment of alginic acid or its salts with monovalent cations, such as Na-alginate, K-alginate with a combination of compounds releasing HCO₃¹⁻ and CO₃²⁻ions, divalent cations such as Ca²⁺, Ba²⁺, Cu²⁺, Sr²⁺, Zn²⁺, Fe²⁺, Fe³⁺ Mn²⁺, Al³⁺, ions, to form gelated particles,
- treatment with the Zr⁴⁺ ions simultaneously with the previous step of gelation or after the gelation to form hydrophobic porous gelated particles, and
- addition of microorganisms and optionally nutrients in the first step or after the second step to obtain hydrophobic porous gelated particles with incorporated microorganisms.

2. The method according to claim 1, wherein divalent Ca ions are used in the first step and CaCO₃, CaCl₂ or any other compound releasing said Ca ions is used as source of said divalent Ca ions.

3. The method according to claim 2, wherein the source of divalent Ca ions is CaCOs and/or 0.1-1 M CaCl₂, wherein CaCOs suspension is preferably 0.1-10 % w/v and comprises particles with size 10 nm - 10 µm.

4. The method according to any of the preceding claims, wherein Zr ions used in the second step originate from ZrOCl₂ or any other compound releasing Zr ions.

5. The method according to any of the preceding claims, wherein microorganisms in suspension are added to the prepared carriers in the second step and vacuum is used to impregnate the carriers with microbes.

6. The method according to the preceding claim, wherein after application of vacuum a further step of treatment with CaCl₂ is performed.

7. The method according to any of the preceding claims, wherein microorganisms are selected in the group comprising fungi, Gram-negative and Gram-positive bacteria, mesophilic microorganisms, thermophilic microorganisms, psychrophilic aerobic microorganisms, anaerobic microorganisms and halotolerant, non-halophiles as well as slight halophile bacteria.

8. The method according to the preceding claim, wherein microorganisms are oil degrading bacterial cells.

9. The method according to any claim from 1 to 8, wherein the method comprises the following steps:
- mixing alginic acid and CaCO₃,
- dropwise adding the mixture obtained in the previous step to ZrOCl₂ to introduce pores and transforming alginate surface from hydrophilic to hydrophobic,
- washing with a buffer that can buffer pH ranges between 4 to 10,
- wherein addition of microorganisms and nutrients can be performed before addition of ZrOCl₂ or after washing with the buffer.

10. The method according to any claim from 1 to 8, wherein the method comprises the following steps:
- mixing alginic acid and CaCO₃,
- dropwise adding the mixture obtained in the previous step into CaCl₂ to obtain gelated alginate particles, for example formed as beads, sheets, threads,
- adding ZrOCl₂ to introduce pores and transforming alginate surface from hydrophilic to hydrophobic,
- washing with a buffer that can buffer pH ranges between 4 to 10,
- wherein addition of microorganisms and nutrients can be performed before addition of CaCl₂ or after washing with the buffer.

11. The method according to any claim from 1 to 8, wherein the method comprises the following steps:
- mixing alginic acid and CaCO₃,
- preparing solid support carriers, combined with a suspension comprising microbial cells, nutrients, alginate and CaCOs for impregnation of porous solid supports, using depressurization,
- gelation of impregnated solid support carriers from the previous step with addition of CaCl₂ solution to gelate alginate that was introduced into the pores of the solid support carriers, washing with water and treatment with 1 % to 10 % solution of ZrOCl₂ to introduce pores and transforming alginate surface from hydrophilic to hydrophobic,
or
the impregnated solid support carriers are directly dropped in or washed in 1 % to 10 % ZrOCl₂ solution to introduce pores and transforming alginate surface from hydrophilic to hydrophobic, and
- washing with a buffer that can buffer pH ranges between 4 to 10

12. The method according to the preceding claim, wherein solid support carriers are selected in the group comprising expanded clay, porous sandstones, vermiculite, straw particles, and cellulose.

13. The method according to any claim from 1 to 8, wherein the method comprises the following steps:
- mixing alginic acid and CaCO₃,
- dropwise adding the mixture obtained in the previous step into CaCl₂ to obtain gelated alginate particles, for example formed as beads, sheets, threads,
- adding ZrOCl₂ to introduce pores and transforming alginate surface from hydrophilic to hydrophobic,
- washing with a buffer that can buffer pH ranges between 4 to 10 to obtain alginate carriers,
- adding microorganisms and nutrients to the alginate carriers prepared in the previous step,
- treatment with vacuum to impregnate the carriers with the microorganisms added in the previous step, and
- optionally adding CaCl₂ for improved gelation.

14. The method according to any claim from 1 to 8, wherein the method comprises the following steps:
- mixing alginic acid and CaCO₃,
- dropwise adding the mixture obtained in the previous step into CaCl₂ to obtain gelated alginate particles, for example formed as beads, sheets, threads,
- adding an acid solution, such as acetic acid, lactic acid, citric acid, orthophosporic acid, or preferably HCl, for acidification,
- adding ZrOCl₂ to introduce pores and transforming alginate surface from hydrophilic to hydrophobic,
- washing with a buffer that can buffer pH ranges between 4 to 10 to obtain washed alginate carriers,
- adding microorganisms and nutrients to the alginate carriers prepared in the previous step,
- treatment with vacuum to impregnate the carriers with the microorganisms added in the previous step, and
- optionally adding CaCl₂ for improved gelation.

15. Hydrophobic porous alginate carriers comprising live bacterial cells prepared according to any of the preceding claims.
